# EUROPEAN PATENT APPLICATION

(11) **EP 1 072 277 A1**
(43) Date of publication of application: **31.01.2001**
(21) Application number: 00306471.4
(22) Date of filing: 28.07.2000
(51) Int. Cl.: A61L 27/16, A61L 2/08

(54) **Gamma irradiated heat treated implant for mechanical strength**

(30) Priority: 29.07.1999 US 362966
(71) Applicant: DEPUY ORTHOPAEDICS, INC., Warsaw, Indiana 46581 (US)
(72) Inventor: Hamilton, John V., Foxborough, Massachusetts 02035 (US); Schmidt, Mary Beth, Pomfret Centre, Connecticut 06259 (US); Greer, Keith, Fort Wayne, IN 46814-3256 (US)
(74) Representative: Belcher, Simon James

(57) **Abstract**

Wear resistance and oxidation resistance of a bioimplantable polymeric prosthesis components are achieved in a radiation cross-linked part by packaging a bulk material, unfinished or finished parts within a gas impermeable package substantially free of oxygen, irradiating the package and contents with penetrating radiation to an extent sufficient to produce a desired substantial level of cross-linking within the polymer, and raising the object to a thermal threshold lying below the polymer melt temperature for a time effective to react released gas and reactive species and stabilize the material against subsequent oxidative reactions, while achieving high breaking elongation. The stabilization process is preferably carried out on packaged components in the presence of gases released during irradiation, which recombine, substantially eliminating free radicals without introducing additional thermal cross-linking. The process does not alter component dimensions, and may be applied to finished components. The procedure achieves high levels of cross-linking while maintaining mechanical strength and reducing susceptibility to aging or chemical degradation.

## Description

### REFERENCE TO RELATED APPLICATIONS

This application is related to the United States patent application entitled **LOW TEMPERATURE PRESSURE STABILIZATION OF IMPLANT COMPONENT** (Attorney Docke No.22675-176) and the United States patent application entitled **TWO STEP GAMMA IRRADIATION OF POLYMERIC BIOIMPLANT** (Attorney Docket No. 22675-178) each filed simultaneously herewith on July 29, 1999 by Express Mail with Express Mail Label No. EJ000510233US and No. EJ0005510255US, respectively, by the present inventors. Each of the foregoing patent applications is hereby incorporated herein by reference in its entirety.

### FIELD OF THE INVENTION

The invention provides a method for increasing the wear resistance of polymeric parts. The method is particularly well suited to polymeric parts that are biocompatible and that are intended for use as components of artificial joints. A variety of polymeric materials, particularly polyolefins and especially ultra high molecular weight polyethylene (UHMWPE), can be treated according to the method of the invention to make articles of improved wear resistance and improved oxidation resistance.

### BACKGROUND OF THE INVENTION

This invention relates to bioimplantable polymeric articles and more particularly to materials treatment methods for improving the wear resistance and oxidation resistance of such articles.

Advances in biomedical engineering have resulted in numerous polymeric articles, particularly load bearing or mechanical articles, which are to be implanted within the body. Polymeric components are widely used in orthopedic surgery, for example, to form articulation surfaces within artificial joints. Ultrahigh molecular weight polyethylene (UHMWPE) is an example of a polymer that is commonly used to form components of artificial joints.

Among the properties required of bioimplantable polymeric components, particularly those used in artificial joints, are low friction, biocompatibility, and good mechanical properties, including hardness, tensile strength and other properties related to toughness and resistance to wear. Such components must also be sterile before implantation within a patient.

Some polymers and medical devices may be adversely affected by heat sterilization, so such a technique is not widely used. Ethylene oxide sterilization is another technique for sterilizing medical devices, but ethylene oxide can pose health and environmental risks that render this method less desirable. As a result, a preferred method of sterilizing many medical devices, including polymeric components, is by exposure to forms of penetrating ionizing radiation, such as gamma ray, x-ray and electron beam radiation.

Presently, sterilization by gamma radiation is a preferred method for sterilizing many medical devices, including bioimplantable polymeric components. However, gamma radiation initiates chemical reactions within the polymer that can affect the structure, morphology and mechanical properties of the polymer. During gamma irradiation a variety of chemical species, such as ions, excited molecules, double bonds, oxidation products and free radicals are created within the polymer. Free radicals are believed to be a species generated during gamma radiation that may contribute most to changes in the properties of irradiated polymers.

Once free radicals are formed within a polymer, these species may participate in at least four major types of reactions. The free radicals can undergo a recombination reaction by reacting with hydrogen to eliminate the free radical, by reacting with carbon molecules to create side chains, or both. Free radicals can also initiate or participate in chain scission reactions that result in a decrease in the molecular weight, and an increase or change in the density and crystallinity, of the polymer, thus causing some mechanical properties of the polymer to degrade. A cross-linking reaction is another reaction in which the free radicals can participate. Finally, the free radicals may remain trapped within a polymeric material for an extended time period (e.g., years) without initially reacting, thus remaining available to react as conditions dictate.

The presence of oxygen in the polymeric material or in its surrounding environment can contribute to an oxidation reaction in which free radicals and dissolved oxygen react to produce a compound with a carbonyl functional group, resulting in chain scission and the creation of new free radicals. Thus, oxidation can decrease the molecular weight of a polymer (due to such chain scission), which in turn, may contribute to the degradation of its mechanical properties. Since oxygen is ubiquitous in the atmosphere and in biological fluids, this mechanism of degradation may occur whenever there remains a substantial concentration of free radicals in the irradiated polymer. The initial sterilization of polymer components by gamma radiation in air is also believed to decrease the wear resistance of polymers due, in part, to such oxidation effects.

Since wear resistance is a key mechanical property for polymeric components that are used in joint prostheses, this problem has now been addressed by techniques such as exposure in oxygen-free environments, by subsequent removal of the affected surface layer, or other processes. Thus, one current practice addresses this problem by irradiating polymeric components in an environment of an inert gas (e.g., argon, helium, nitrogen) to minimize oxidation effects. *See*, Kurth, M. et al., *"Effects of Radiation Sterilization on UHMW-Polyethylene"* Antec 87, pp. 1193-1197(1987); Streicher, R.K., *Radiol. Phys. Chem.*, Vol. 31, Nos. 4-6, pp. 693-698 (1988); Streicher, R.M., "Improving UHMWPE by Ionizing Radiation Cross linking During Sterilization", 17th Annual Meeting of the Society for BioMaterials, p. 181 (1991). Others have used vacuum techniques to help purge an environment of oxygen before conducting gamma radiation sterilization. See, Yong Zhao, et al., *J. Appl. Polymer Sci.*, Vol. 50, pp. 1797-1801 (1993), and Hamilton, U.S. patent 5,577,368.

Natural friction and aging of a replaced, artificial joint can cause minute particles of debris (e.g., particles from a polymeric component) to become dislodged and to migrate within the joint. This phenomenon of wear debris within artificial joints is a serious problem that can inhibit the proper mechanical functioning of the joint. Wear debris can also lead to osteolysis and bone deterioration. If osteolysis develops around an artificial joint, correction typically requires surgical removal of the diseased tissue and revision of the artificial joint.

Resistance to such wear depends on many factors, such as the hardness and toughness of the polymer material, which may in turn depend upon specific physico-chemical characteristics such as the molecular weight of the resins, the degree of cross linking of the material, and the relative amounts and distribution of amorphous and of crystalline polymer. Each of these basic characteristics of the material may be affected by the application of radiation, heat or chemical agents. Moreover, the fabrication of polymeric components generally proceeds from a starting material, or resin, which is provided as either a powder or granular material, or as a consolidated blank, e.g., a sheet or block or preform made from the resin, which must be machined to final form. Heat, other conditions, or materials involved in the consolidation step may also influence underlying physical properties, and cross-linking irradiation may be necessary to form the resin material into a sufficiently wear-resistant solid article. Moreover, when cross-linking by ionizing radiation, different forms of irradiation may each have different absorption or interaction characteristics, or generate heat which further affects the crystallinity and other properties. Thus, wear resistance depends in a rather complex way upon a number of processing steps which may be employed to treat or physically form the polymer component.

Workers in the field have developed several approaches to processing the polymer material or finished parts in attempts to introduce effective levels of cross-linking while attaining suitable materials properties, or rectifying processing damage. These approaches include irradiation in a vacuum, cold irradiation with subsequent melting of bulk material, chemical cross-linking, and gas plasma treatment, among others. Each of these techniques appears to enhance at least one parameter. However, the need for multi-step processing, the number of related and often competing physical effects involved, and the empirical nature of evaluating actual long-term changes in the prosthesis strength and wear properties, all result in a continuing need for manufacturing processes of polymer prosthetic components that are effective to enhance the polymer strength and resistance to wear.

Because strength and wear resistance are properties of such importance for polymeric components used to form artificial joints, it would be advantageous to provide sterilized polymer components that have a desired degree of cross-linking for wear resistance while not impairing their strength or introducing long-term aging effects.

It is thus an object of the invention to provide methods for increasing the wear resistance of bioimplantable polymeric components.

It is also an object to provide cross-linking or sterilization techniques for medical grade implantable polymer components that impart or preserve important properties of the components.

A further object is to provide bioimplantable polymeric components that have excellent elongation strength and that are less prone to the effects of oxidation.

These and other objects will be apparent to one of ordinary skill in the art upon reading the description that follows.

### SUMMARY OF THE INVENTION

According to the method of the invention, bulk polymeric material or a finished or unfinished component for forming a bioimplantable prosthesis, is placed within a gas impermeable package or container which is evacuated and sealed so that the contents are maintained in isolation and under a vacuum, for example, of about 20 mbar. Next, the package and contents are irradiated with a relatively high dose of penetrating radiation calculated to achieve a target level of cross linking of the polymer that forms the part. Gamma radiation using a Cobalt-60 source to provide the gamma radiation is a preferred radiation technique.

The bulk material or component remains in the packaging, and it is then heated to a cure temperature that is elevated, but well below its melt point, for example between about 30° - 140°C, preferably to about 110°C, and is held at that temperature for a time to effectively to eliminate free radicals created by the irradiation and thus stabilize the treated material. During the initial irradiation, hydrogen is released by the various chemical changes that occur in the polymer. Evolved gases are retained in the vacuum package, and the material or component remains bathed in the hydrogen during heat cure treatment so that during the beat stabilization stage the hydrogen terminates immobilized free radicals. The packaged material is then slowly cooled, e.g., at a rate of about 5°C/hr. The level of cure heating is sufficiently below the melt temperature of the resin to avoid the immediate weakening of mechanical properties that would result from excessive melt/recrystallization, while it is sufficiently high to promote molecular motion and gas diffusion such that the desired stabilization occurs within about twelve hours. The combined process decouples the cross-linking and stabilization processes in a manner that allows a high level of effective cross-linking and subsequent stabilization without impairing mechanical strength.

Applicant has found that when components are subjected to the foregoing treatment, it is possible to attain the target level of cross-linking together with a low peak oxidation index through the wear depth of the component. In addition, elongation strength is unimpaired, and is substantial and suitable for a variety of prosthetic uses, while maintaining dimensional stability. The process may be applied to fully finished components. The process may also be applied to bulk material or unfinished components, in which case the material or components are next removed from their packages, and machined to form finished components of a prosthesis. The finished components are then sterilized and packaged.

A benefit of the invention is the improved ability of the polymeric parts to resist oxidation while attaining both a high level of cross-linking and mechanical toughness. The resulting sterilized part is characterized by a cross-linked material of substantially reduced oxidation index extending with a depth profile to well over a millimeter that provide sustained wearability and oxidation resistance in prolonged use, and yet having a high elongation strength.

### BRIEF DESCRIPTION OF THE DRAWINGS

Figure 1 is a graph which plots distribution of oxidation index for the invention and two sample processes described in Example 1;
Figure 2 is a chart illustrating cross-linking achieved by the invention; and
Figure 3 illustrates elongation test results achieved by the invention.

### DETAILED DESCRIPTION OF THE INVENTION

The present invention provides a method for improving the wear resistance of manufactured polymeric parts. According to this process the material for desired polymeric part is first consolidated from a resin or powder, and/or is formed into either a finished component, or a bulk or rough blank, or an unfinished part by one or more known processes, such as compression molding, injection molding, ram extrusion and machining. The polymer that forms the part can be low density polyethylene, high density polyethylene, ultrahigh molecular weight polyethylene (UHMWPE), polypropylene, polyester, nylon, polyurethane, poly(methylmethacrylate), or other biocompatible polymer typically used in biomedical applications. Preferred polymers are polyolefins, and particularly UHMWPE, which is commonly used in a variety of orthopedic implants, such as liners for acetabular shells of artificial hip joints, and as tibial bearing members in artificial knee joints. The invention will now be described with reference to a UHMWPE component. The UHMWPE material is available from a number of suppliers as a resin, typically a powder or granular material, or a sheet or bar for various working or forming steps. The techniques for forming this material are well known, and will not be further described here.

In one embodiment, the finished component, or the bulk polymeric material or an unfinished part, that is to be subjected to the process of the invention is first placed in an isolation compartment such as a laminated flexible package that is formed with a gas-impermeable film and has a heat sealable opening therein. The bulk material may take the form of sheets, pucks, rods, bars, billets or other common shape resulting from or following the consolidation process utilized on a starting resin. An unfinished part may, for example, constitute a compression molded oversize blank, or a partially finished component machined from a previously consolidated blank or bulk material. When a bulk blank or unfinished part is used, it may be subjected to precision shaping or finishing after carrying out the first steps of the invention. This starting part or material, either finished, unfinished or bulk, will henceforth be referred to, interchangeably, as either the polymer object or the component. More than one polymer object can be placed in a single package. The package and the object are then subjected to a vacuum while the heat sealable opening remains open and the vacuum is maintained for a time effective to allow outgassing of absorbed gases prior to sealing. Thereafter, the package is heat sealed while maintaining the vacuum, thus closing the component in an evacuated environment. Preferably a vacuum of 0-50 mbar, and preferably under 20 mbar is applied, so that the package interior is essentially free of oxygen and other adverse gases.

Thus, the packaging of the polymer object in a heat sealed package under vacuum is carried out to reduce pressure within the package and to remove absorbed oxygen from the polymer material and maintain an oxygen-free environment within the package. The package preferably includes one or more barrier layers that are effective to render it impermeable to gaseous hydrogen, so that hydrogen released during radiation treatment will remain within the package.

Following vacuum heat sealing of the package that contains the polymer object, the package and the object are irradiated with penetrating radiation for a period of time that is sufficient to introduce a substantial level of cross-linking of polymer chains of the polymer material. Various acceptable forms of ionizing radiation can be used to effect the sterilization of the part. These radiation forms include gamma rays, x-rays, and electron beam radiation. Gamma radiation effectively penetrates packaged material and is currently a preferred irradiation technique. The dose is selected to achieve a target level of cross-linking. For a UHMWPE component, the dose is selected to be high enough, for example, to achieve cross-linking at least comparable to that of a current commercial prosthesis. A generally useful range in existing products is around 40 kGy, and it is a feature of the present invention that substantially higher levels of radiation may be applied, and a substantially greater degree of cross-linking achieved, without several of the deleterious effects normally associated with the higher doses. This may be in the range of 60-120 kGy, and preferably about 60-80 kGy.

The flexible packaging material is a high barrier flexible packaging material of the type commonly used to enclose medical devices. Preferably the packaging material is a multilayered, heat seal peelable packaging material that is impermeable to gas, including hydrogen, and includes a barrier layer, various polymer layers and a heat seal coating. Examples of suitable materials are those that include the following layers: polyester film-low density polyethylene-foil-ionomer-heat seal coating. Packaging materials having the following layers can also be used: polyester-low density polyethylene-foil-EAA-linear low density polyethylene-heat seal coating; and polyester-Surlyn-nylon-Surlyn-foil-EAA-linear low density polyethylene-heat seal coating. Suitable packaging materials can be obtained from a variety of sources, including Tolas Health Care Packaging of Feasterville, Pennsylvania. The thickness of the packaging material preferably is in the range of about 2 mils to 7 mils (50-175 micrometers). While the invention may be practiced using hard vessels as containers during processing, the use of vacuum foil bags to contain the polymer objects offer significant advantages in terms of process handling and simplicity. Furthermore, it locally retains a relatively high concentration of radiation-released or self-generated hydrogen, as discussed below, resulting in a simple, safe and effective stabilization protocol.

According to the process of the invention, a relatively strong vacuum is used which is sufficient to remove all or substantially all oxygen from within the package and the adjacent environment. A vacuum in the range of approximately 0-50 mbar and preferably under about 20 mbar is employed, and is maintained about the package and the polymeric material or component for a time effective to substantially dispel residual or surface-adhered oxygen, before sealing. While the vacuum is maintained, the package is heat scaled, rendering it both evacuated and impermeable to gases. Techniques for vacuum heat sealing such packaging material are readily available. Suitable packaging equipment for heat sealing packages under vacuum will be known to those of ordinary skill in the art. An example of a suitable vacuum packaging apparatus is a Multi Vac A342 apparatus, available from Multivac, Inc. of Kansas City, Missouri. Optionally, the package may be backfilled with an inert gas, such as nitrogen or a noble gas. However, the cross-linking step evolves additional gases that remain within the package so it is generally preferred to seal the evacuated package, rather than backfilling and scaling at this stage, so that after irradiation, the package interior holds only a small volume of gas, and this is primarily those gases released by the component.

As noted above, the packaged polymeric material is irradiated using penetrating radiation, which in the prototype process is gamma radiation having an energy of about 1.25 MeV, such as radiation from a source such as a Cobalt 60 source. The dose of gamma radiation is administered to introduce or initiate a substantial level of cross-linking, and is typically administered during an exposure lasting several hours or more. As applied to an UHMWPE material such as GUR 1020 or GUR 1050 resin, applicant contemplates a dosage which is several times greater than a sterilizing dose, or approximately 60-120 kGy for a prosthetic component, and approximately 60-90 kGy is presently preferred.

Thus, the polymer material is placed in an environment free of oxygen, and irradiated with penetrating radiation to induce a substantial or effective level of cross-linking. A dose of 40 kGy has been found useful in some prior art processes to introduce a reasonably effective level of cross linking in a GUR 1020 material, but the range of radiative cross-linking has been limited by the fact that higher doses are associated with degradation of tensile properties or decreased oxidation resistance. The aim of the present invention is to introduce a noticeably higher level of cross-linking but without impairing certain necessary characteristics, so that the resistance to wear is improved. Applicant refers to this higher level of cross-linking as an "effective level". As noted above, the irradiation process impairs other desired properties, so it has previously been necessary for the level of cross-linking applied in existing UHMWPE articles to be set lower than an optimum level, in order not to sacrifice other properties necessary to the prosthesis strength or wear resistance. As described in greater detail below, the present process overcomes these collateral effects, so that, for example, by irradiating to achieve greater cross linking than that of a current product level, greater hardness is achieved while the final collateral properties will nonetheless equal or exceed that of the existing product. Thus, where a prior art process may utilize a cross-linking dose of 40 kGy, the present invention may, for example, irradiate the packaged polymer object with a dose of 50-60 kGy or even 80 kGy to attain a much greater degree of cross-linking while also achieving high breaking elongation, a critical mechanical property. This combination with a higher degree of cross-linking is effective to significantly reduce wear.

Continuing now with a description of the process, following irradiation, the polymer object is heated but kept to a temperature well below its melt point. For example, it may be heated to approximately 110°C for UHMWPE material while still in sealed packages in which it was irradiated. This limited elevated heating promotes flexing and kinetic mobility of long chain molecules, as well as enhanced diffusion of trapped or dissolved hydrogen without, however, allowing the bulk material to fuse, deform or recrystallize. These two factors allow isolated or immobilized free radicals to recombine, so that in time they are either terminated or form further inter-strand cross links. In one prototype implementation, the objects were heated in their isolation containers for twelve hours, and the extended heating was effective to substantially eliminate the free radicals formed by the initial dose of radiation reducing the residual activity to a negligible level. The polymer was then slowly cooled from the cure temperature, at a rate of approximately 5°C/hour.

The foregoing process of substantial cross-linking followed by controlled thermal elevation for an extended time does not introduce deformation or substantial changes of dimension in the treated polymer, and it results in a material or component which is uniformly and highly cross-linked, and has also been stabilized so that it does not contain a significant level of free radicals and is not prone to degrade when subsequently stored or exposed to oxygenated environments over time. Furthermore, applicant has found this treatment to result in a material having a high breaking elongation over 300% - resulting in high mechanical toughness.

### EXAMPLE

Solid sample articles were formed from a standard grade of medical UHMWPE, using either a GUR 1020 or GUR 1050 resin. These articles were placed in aluminum foil vacuum package bags, evacuated, and sealed at a pressure of approximately 18 mbar. The sealed articles were irradiated with a dose of approximately 80 kGy of gamma radiation at an energy of approximately 1.25 MeV, then heated in the packages and maintained at a temperature of 110°C for twelve hours. After slow cooling at a rate of 5°C/hr, the articles were removed from the packages and the cooled treated articles were tested to determine their oxidation index, degree of cross-linking and one or more basic mechanical properties.

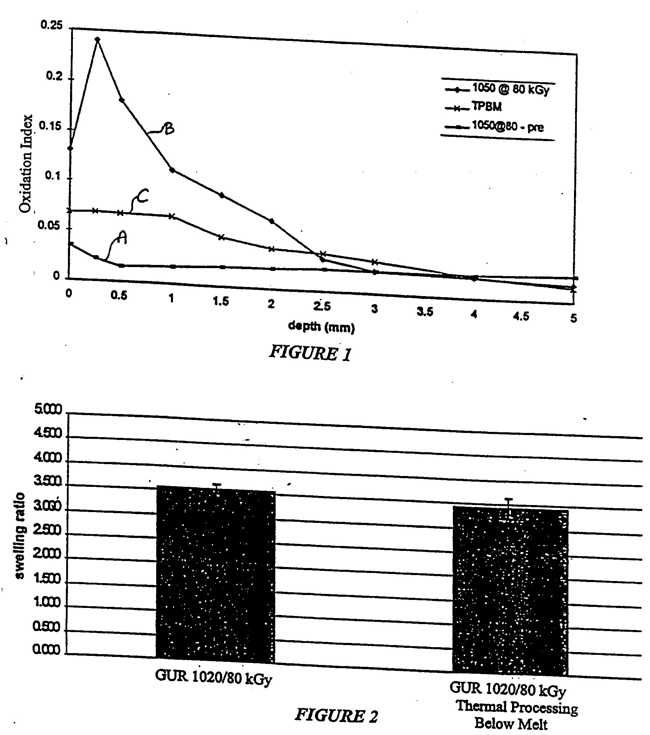

The oxidation index was measured after an oxidative challenge of the article by taking measurements on a section at a range of depths, and this was compared to measurements taken before and after oxidative challenge on samples exposed to the same dose of cross-linking radiation. Figure 1 graphs the observed oxidation index for GUR 1050 resin material cross-linked at the 80 kGy dose before oxidative challenge (Curve A), and after challenge (Curve B). Curve C shows the after-challenge oxidation index for the same resin and dose when heat treated below its melt temperature in accordance with the present invention.

Cross-linking of the treated articles was evaluated by determining swelling ratio, and this was compared to that of a baseline sample article formed of the same resin and subjected to the same irradiation level but with no subsequent thermal treatment. Figure 2 illustrates the results of this comparison for a GUR 1020 material cross-linked with a dose of 80 kGy.

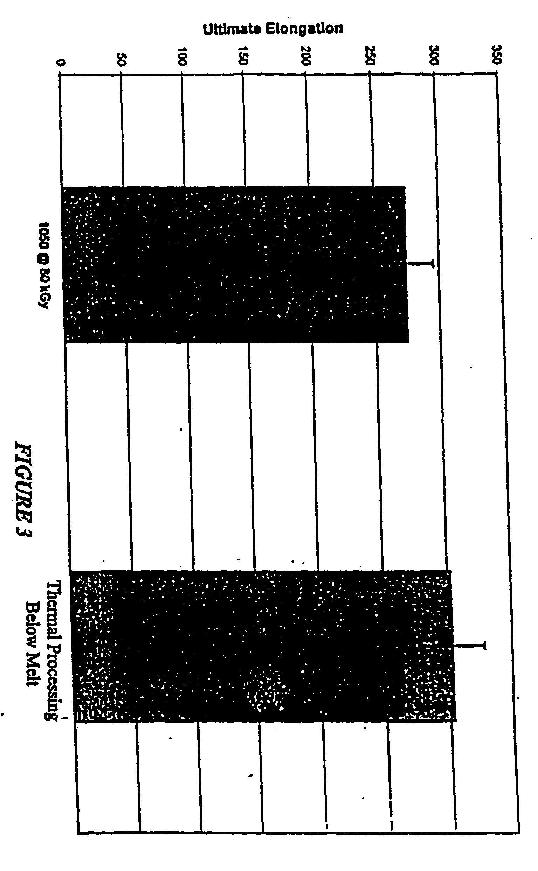

The elongation at failure was evaluated by standard testing techniques (e.g., ASTM specification F648) and was compared to that of a sample formed of GUR 1050 material and exposed to the same irradiation. The results are shown in Figure 3.

### Discussion

As shown in Figure 1, the thermal processing below melt was effective to substantially reduce the susceptibility to oxidation, with the oxidation index OI lying well below that of the irradiated but not thermally cured material for a depth of over two millimeters in the surface region. This indicates that the thermal stabilization was effective to substantially reduce the level of residual free radicals introduced by the high radiation dose, eliminating the principal cause of long-term material instability.

Figure 2 shows that the change in swelling ratio due to the thermal cure of Example 1 was not measurably significant, indicating that the thermal processing at this temperature produced little, or no additional cross-linking. Thus, dimensions were stable, and the level of cross-linking was directly determined by the dose of radiation.

Figure 3 shows that an ultimate elongation over 300% was achieved with the processing of the invention, a remarkably high level for the 80 kGy radiation dose, and an increase in elongation of about ten percent over that of a GUR 1050 material treated with a dose of 80 kGy. This elongation strength coupled with the high degree of cross-linking offers a significant improvement in toughness and wear resistance.

As noted above, in the prior art, the process of irradiation introduces instabilities and bond breakage that are generally addressed by stabilization using one or more secondary processes of melting and chemical treatment, possibly under high pressure conditions, and these in turn may alter a complete matrix of essential physical characteristics, either immediately or in the long term, affecting strength and wear. EXAMPLE 1 demonstrates that a high and desirable level of cross-linking may be achieved without sacrificing critical breaking strength by irradiating and curing in sealed containers, where the curing is carried out by warming to enhance molecular kinetics and hydrogen mobility, but at a temperature sufficiently below melt temperature so that destructive or somewhat unpredictable changes of melting and recrystallization do not impair mechanical strength. In general, the curing is carried out for a time, on the order of four hours to about five days, that is effective to stabilize the radiation damage and, as indicated in Example 1, under thermal conditions effective to preserve or enhance mechanical strength.

Overall dimensions of the part remain the same, without deformation, making the process suitable for direct application to finished components in addition to bulk material or unfinished parts. Advantageously, the low peak oxidative index shown in Figure 1 for a component irradiated at 80 kGy with this process lies below that of a standard component irradiated at 40 kGy in gas vacuum foil packaging. Thus, the level of cross-linking is decoupled from several competing processes typically connected with stabilization that may otherwise impair the strength and durability of the prosthesis.

Thus, rather than having the achievable level of cross-linking be limited by increasing levels of adverse side effects, the level of radiation may be set to directly achieve the high desired level of cross-linking as shown in Figure 1, and the material is then subsequently stabilized without introducing any significant further cross-linking or material deformation. This contrasts sharply with cross-linking processes of the prior art that may introduce increasingly high levels of unwanted side effects or require additional preparation steps to achieve the desired level of cross-linking.

The process also presents significant manufacturing benefits, in that, when performed on finished prosthetic joint components in vacuum bags, the components may be cross-linked, sterilized, and stabilized simultaneously or without removal from their packages. In this case, the thermal cure temperature must not be set so high as to impair integrity of the sealed packages. The process may also be applied to unfinished material or components, in which case somewhat higher temperatures may be employed since the treated parts will be later removed from their packages for further machining.

Alternatively, the packaging and irradiation steps may be implemented by placing the polymer object in either a rigid or a flexible container. All or most of the oxygen is first evacuated from the container. Optionally, the container may next be pressurized with an inert gas (e.g. argon, helium or nitrogen) to a pressure between approximately one-tenth and several atmospheres. Subsequently, the container and the enclosed polymeric parts are irradiated as described above to an extent sufficient to promote the aforesaid substantial level of cross-linking of the polymer. A variation of this embodiment dispenses with the need to evacuate the container by flushing it with a sufficient amount of an inert gas to displace any oxygen before irradiating the polymer object. When such a chamber is utilized instead of vacuum bag packaging, a more elevated cure temperature may be employed without affecting chamber integrity.

The foregoing description of the method of manufacture and the illustrative embodiments is presented to indicate the range of constructions to which the invention applies. Variations in the materials to be used to fabricate polymer samples, vacuum pressures, radiation sources, and the like, will be readily apparent to those having ordinary skill in the art. Such variations are considered to be within the scope of the invention in which patent rights are asserted, as set forth in the claims appended hereto.

The entirety of all publications and/or references noted herein are expressly incorporated by reference herein.

## Claims

1. A method of preparing of a prosthesis wear surface component formed of an irradiation cross-linkable polymer material, such method comprising the steps of
providing a wear surface blank formed of polymeric material
exposing the blank in a sealed oxygen-free package to a dose of gamma radiation effective to substantially introduce an effective target level of cross-linking in said polymer material, and
elevating the temperature of the exposed blank to an elevated thermal threshold lying below its melt temperature while the blank remains in the sealed package and maintaining said elevated thermal threshold for a time effective to
i) reduce residual free radicals and stabilize said polymer material so as to enhance resistance to oxidative degradation over time, and
ii) keep the level of cross-linking substantially unchanged.

2. The method of claim 1, wherein the step of providing a wear surface blank is performed by providing a finished component.

3. The method of claim 1, wherein the polymer material is a polyolefin.

4. The method of claim 3, wherein the polymer material is UHMWPE, and said thermal threshold is a temperature under about 120°C.

5. The method of claim 1, further comprising the step of slowly cooling to ambient.

6. The method of claim 3, wherein the dose of gamma radiation is between 50 and 100 kGy.

7. The method of claim 4, wherein the dose of gamma radiation is approximately 80 kGy.

8. The method of claim 4, wherein the step of exposing is effected by vacuum foil packaging and exposing in the package so that the package retains gases evolved during exposure.

9. A prosthesis component formed of a polymeric material and having a wear surface, said component having a near surface region extending for a depth below said wear surface, wherein said component is an ultra high molecular weight polyethylene (UHMWPE) component cross linked by radiation and characterized in that said near surface region has a degree of cross-linking characteristic of irradiation at a dose above 50 kGy, and a breaking elongation of about 250% or more effective to impart wear resistance for weight-bearing articulation of the prosthesis, and has an oxidation index after challenge that lies below 0.10 for a depth of at least a millimeter in said near surface region.

10. An implantable prosthesis polymeric wear surface component having enhanced resistance to degradation, such component comprising
irradiated blank or finished prosthesis component being irradiated to introduce an effective level of cross-linking for hardness
and being warmed and thermally cured at a temperature below its melt temperature for a time effective to terminate reactive species therein and stabilize the component against oxidation while achieving a breaking elongation above about 250% thereby providing toughness to impart improved wear resistance for articulation.

11. An implantable prosthesis polymeric wear surface component having enhanced resistance to degradation, such component comprising an irradiated blank or finished prosthesis component being irradiated to introduce a desired level of cross-linking while evolving radiation damage products
and being thermally cured below its melt temperature in the presence of said damage products and for a time effective to react trapped species in the blank and stabilize the blank against oxidation while enhancing elongation strength.

12. A method of preparing of a prosthesis wear surface component formed of an irradiation cross-linkable polymer material, such method comprising the steps of
providing a wear surface blank formed of polymeric material
exposing the blank in a sealed oxygen-free package to a dose of gamma radiation effective to substantially introduce an effective target level of cross-linking in said polymer material, and
raising the temperature of the exposed blank to an elevated thermal threshold lying below its melt temperature while the blank remains in the sealed package and maintaining said elevated thermal threshold for a time effective to
i) reduce residual free radicals and stabilize said polymer material so as to enhance resistance to oxidative degradation over time, and
ii) enhance the breaking elongation of the material
thereby improving toughness and wear resistance.
